# EUROPÄISCHE PATENTSCHRIFT

(11) **EP 0 480 325 B1**
(45) Veröffentlichungstag und Bekanntmachung des Hinweises auf die Patenterteilung: **17.04.1996**
(21) Anmeldenummer: 91116954.8
(22) Anmeldetag: 04.10.1991
(51) Int. Cl.: G01N 33/18, G01N 21/64, C02F 3/00, G01N 33/22

(54) **Verfahren zum Bestimmen des chemischen Sauerstoff-Bedarfs (CSB)**
Method for determining the chemical oxygen demand (cod)
Procédé de détermination de la demande chimique en oxygène (dco)

(30) Priorität: 06.10.1990 DE 4031727
(43) Veröffentlichungstag der Anmeldung: 15.04.1992
(73) Patentinhaber: Böhler, Kurt, D-76275 Ettlingen (DE)
(72) Erfinder: Böhler, Kurt, D-76275 Ettlingen (DE)
(74) Vertreter: Trappenberg, Hans

(56) Entgegenhaltungen:
- EP-A- 0 282 441
- EP-A- 0 427 996
- DE-A- 3 811 540

## Beschreibung

Die Erfindung betrifft ein Verfahren zum Bestimmen des Chemischen Sauerstoff-Bedarfs (CSB) eines Mischabwassers mit einer Schmutzfracht aus organischen und anorganischen Substanzen durch Nachweis des verbleibenden CSB nach dem Durchlauf durch eine chemisch/physikalische und/oder biologische Abwasser-Reinigungsanlage.

Zum Bestimmen der in einem Abwasser mitgeführten organischen Schmutzstoffe ist seit langem schon die Messung des Biochemischen Sauerstoff-Bedarfs üblich. Dieser Sauerstoffbedarf zeigt den Verlauf der oxidativen Umwandlung der organischen Schmutzstoffe durch Bakterien innerhalb einer bestimmten Zeit an. Aus praktischen Gründen hat man sich hierbei für die Bestimmung dieses Biochemischen Sauerstoff-Bedarfs nach 5 Tagen bei 20 °C (BSB₅) entschieden. Mit Hilfe dieser seit langem angewendeten Meßmethode und der dafür entwickelten Meßverfahren und -Einrichtungen gelingt es, kontinuierlich den Anteil einer im Abwasser mitgeführten organischen Schmutzfracht zu ermitteln.

Mehr und mehr werden in dem Abwasser aber auch organische und anorganische Stoffe mitgeführt, die den bisher angewendeten biologischen Prozessen nicht zugänglich sind, die also durch die bisher eingesetzten Mikroorganismen nicht abgebaut werden. Stand der Technik ist es, diese bisher dem biologischen Abbau nicht zugänglichen Stoffe durch Zugabe bestimmter Chemikalien zu oxidieren. Hieraus ergibt sich eine neue Bestimmungsgröße, der CSB, als Kenngröße für die Gewässerqualität beziehungsweise die Belastung eines Abwassers. Hierbei ist der ermittelte CSB im allgemeinen stets höher als der BSB, da durch die Chemikalienbeigabe selbstverständlich alle zu oxidierenden Stoffe erfaßt werden, also auch die Stoffe, die biologisch erfaßt werden, also auch die Stoffe, die biologisch abbaubar sind. Hieraus erklärt sich auch, daß der chemische Sauerstoffbedarf stets höher ist als der biologische Sauerstoffbedarf, da bei der Kenngröße CSB sämtliche oxidierbaren Stoffe erfaßt werden, während der BSB nur die biologisch abbaubaren Stoffe berücksichtigt. Dies führt zu einer neuen Kennzahl CSB/BSB, die das Verhältnis der nur chemisch abbaubaren Schmutzfracht zu der biologisch abbaubaren Schmutzfracht anzeigt. Bei häuslichem Abwasser liegt diese Kennzahl etwa bei 2, kann jedoch bei Industrieabwässern durchaus auch über 5 liegen.

Statt der Zugabe von Chemikalien können auch, nach dem heutigen Stand der Technik , speziell gezüchtete Bakterienstämme dem Abwasser zugefügt werden, die diese zuvor nicht abbaubaren Stoffe wiederum biologisch abbauen. Bei diesen schwierig abbaubaren Stoffen handelt es sich insbesondere um Mineralöle, chlorierte Kohlenwasserstoffe, Fette, Zellulose und Tenside. Allerdings müssen diese speziellen Bakterienstämme stets, wie auch zuvor die Chemikalien, dem Abwasser beigegeben werden. Trotz dieser unterschiedlichen Abbauweise wurde lange Zeit das Abwasser nur hinsichtlich seines BSB₅ bewertet, obwohl dieser Biochemische Sauerstoff-Bedarf lediglich die biologisch abbaubare, organische Schmutzfracht berücksichtigt. Dadurch durchliefen des öfteren nicht abgebaute Stoffe die Reinigungsanlage und kamen in mehr oder minder gleichbleibender Form in den Vorfluter und damit in die Flüsse. Erst als die CSB-Belastung überhand nahm, wurde für Gewerbebetriebe, die derartige Belastungen einleiteten, auch eine Messung des Chemischen Sauerstoff-Bedarfs (CSB) vorgeschrieben. Da jedoch ein einheitliches Meßverfahren für diesen CSB nicht existiert, mußte dieser Chemische Sauerstoff-Bedarf stets im Einzelfalle durch entsprechende Probenahme festgestellt werden. Tatsächlich existieren auch heute noch keine Meßeinrichtungen, die kontinuierlich den oxidativen Abbau mancher Schmutzstoffe, insbesondere von Kohlenwasserstoffverbindungen, anzeigen vgl. EP-A-0 282 441 und DE-A-3 811 540, so daß der Nachweis des Abbaus dieser Stoffe noch stets im Labor durch Feststellen des verbleibenden Anteils erfolgen muß. Diese diskontinuierliche CSB-Bestimmung hat selbstverständlich große Nachteile. So kann eine Momentanmessung viel zu hoch oder auch viel zu niedrig ausfallen, obwohl das Abwasser tatsächlich in den durch Gesetze und Verordnungen bestimmten Grenzen belastet war. Die sich dadurch ergebende Unsicherheit führt letztlich zu immer schärferen Bestimmungen beziehungsweise auch zur Aufgabe mancher Produktionen wie auch zu deren Auslagerung in das Ausland, da die zwischenzeitlich erlasssenen Bestimmungen durch Fehlen geeigneter Meßeinrichtungen einfach nicht bestimmbar und damit nicht einhaltbar waren.

Dieses Problem ist schon längere Zeit bekannt, wie es auch bekannt ist, daß es nur durch eine kontinuierliche CSB-Bestimmung gelöst werden kann. Zwar sind bereits Meßeinrichtungen für derartige kontinuierliche Bestimmungen von chemischen Substanzen im Abwasser bekannt, aber jeweils nur für ganz bestimmte chemische Substanzen. Die Vielzahl der verschmutzenden Substanzen jedoch widersetzt sich einer kontinuierlichen Bestimmung und damit auch der kontinuierlichen Bestimmung ihres oxidativen Abbaus im Abwasser. Zwar sind die Bakterienstämme, die diesen oxidativen Abbau der jeweiligen Stoffe herbeiführen, bekannt, jedoch fehlt es an der jeweils notwendigen Bestimmung der quantitativen Zugabe. Zu große oder zu kleine Zugaben dieser speziellen Bakterienstämme führen jedoch dazu, daß entweder die Reinigungsleistung einer gesamten Abwasser-Reinigungsanlage vermindert, teilweise sogar aufgehoben wird, oder dazu, daß im gereinigten Abwasser noch zu viele nicht abgebaute Stoffe vorhanden sind. In vielen Fällen hat man sich daher dazu entschlossen, auf den biologischen Abbau der mitgeführten Stoffe zu verzichten und versucht sie chemisch, beispielsweise durch Zugabe von Kaliumpermanganat, zu oxidieren. Dies führt allerdings dazu, daß diese Chemikalien nicht abgebaut werden, sondern letztlich im Klärschlamm wieder auftauchen.

Aufgabe der Erfindung ist es daher, ein Verfahren zum kontinuierlichen Bestimmen des Chemischen Sauerstoff-Bedarfs (CSB) eines Abwassers anzugeben. Erreicht wird dies durch die Merkmale des Anspruch 1. In erfindungsgemäßer Weise wird durch den Nachweis des Abbaus von einer oder mehreren, im biologischen Abbauverhalten weitgehend mit den im Rohabwasser vorhandenen Stoffen korrelierenden, durch bekannte Meßverfahren hinsichtlich des CSB kontinuierlich erfaßbaren Indikatorsubstanzen, der CSB bestimmt.

Nach der Erfindung wird also nicht mehr direkt der Abbau der eingeleiteten Stoffe bestimmt, sondern lediglich der Abbau einer Indikatorsubstanz, selbstverständlich einer Indikatorsubstanz, deren Vorhandensein durch an sich bekannte Meßverfahren und -Einrichtungen quantitativ erfaßbar ist. Diese Indikatorsubstanz kann bereits im Rohabwasser vorliegen oder kann auch dem Rohabwasser zugegeben werden. Da die CSB-Belastungen insbesondere durch Kohlenwasserstoffe erfolgen, wird als Indikatorsubstanz auch vorzugsweise eine Kohlenwasserstoff-Verbindung, mit Vorteil eine Aminocarbonsäure eingesetzt.

Das Meßverfahren läuft hierbei wie folgt ab:
Ist im Rohabwasser bereits eine im Abbauverhalten weitgehend den gesamten im Rohabwasser vorhandenen Stoffen entsprechende Indikatorsubstanz vorhanden, so kann das Meßverfahren beziehungsweise die Meßeinrichtung auf diese Substanz eingestellt werden. Sollte sich eine solche Substanz nicht bereits im Rohabwasser befinden, ist sie in einer bestimmten Menge beizufügen.

Nach Durchlauf des Rohabwassers durch die Abwasser-Reinigungsanlage unter Beifügung der zum oxidativen Abbau notwendigen speziellen Bakterienstämme werden labormäßig einmalig sowohl der im Abwasser noch vorhandenen CSB wie auch getrennt der CSB der Indikatorsubstanz quantitativ bestimmt und daraus eine Verhältniszahl errechnet, in der der Abbau der verschmutzenden Stoffe im Verhältnis zum Abbau der Indikatorsubstanz steht. Ist diese Verhältniszahl zu groß, liegt also der Abbau der Indikatorsubstanz zu weit ab vom Abbau des CSB der Gesamtschmutzfracht, so ist eine im Abbauverhalten näherliegende Indikatorsubstanz einzusetzen. Im allgemeinen sollte diese Verhältniszahl zwischen 1:0,5 und 1:2, vorteilhafterweise bei 1:1, liegen. Liegt diese Verhältniszahl, also die Korrelation zwischen dem Abbau beziehungsweise der Oxidation der Indikatorsubstanz und demjenigen der gesamten Schmutzfracht, fest, so kann der Abbau der Indikatorsubstanz wie vorgesehen kontinuierlich gemessen und durch Multiplikation mit der Verhältniszahl auch der Abbau der restlichen Stoffe bestimmt werden. Damit ist dann nicht nur der zulässige oder nicht zulässige Anteil der nicht abgebauten Stoffe im gereinigten Abwasser feststellbar, sondern auch erstmals eine Regelung der Zugabe der jeweiligen Hilfsmitteln beziehungsweise Bakterienstämme zur Reinigung des Abwassers, so, daß weder zu wenig noch zu viel dieser Mittel beziehungsweise Bakterien zugegeben werden, möglich. Dadurch ergeben sich nicht nur optimale Werte hinsichtlich des gereinigten Abwassers, sondern auch die Möglichkeit des sofortigen Eingreifens bei Überschreiten des zulässigen CSB wie auch der Nachweis der betrieblichen Sorgfaltspflicht gegenüber dem Gesetzgeber. Damit ist aber auch die Möglichkeit gegeben, Produktionen aufzunehmen, die mit Stoffen arbeiten, die zuvor in ihrem Abbauverhalten nicht oder nur auf die oben beschriebene diskontinuierliche Weise bestimmbar waren.

Aus der Beschreibung des Verfahrens ist ersichtlich, daß es nur dann zuverlässig angewendet werden kann, wenn im Rohabwasser stets etwa die gleichen organischen und anorganischen verschmutzenden Stoffe, sowohl hinsichtlich Menge wie auch hinsichtlich der Art, innerhalb einer gewissen Bandbreite zugeführt werden. Werden in der ursprünglichen Vergleichsmessung nicht enthaltene Stoffe hinzugefügt oder werden die dem Rohabwasser beigegebenen Schmutzmengen stark verändert, kann eine nochmalige Vergleichsmessung notwendig werden. Die Bandbreite hinsichtlich der eingeleiteten Schmutzmengen und der Art der Schmutzstoffe vergrößert sich jedoch deutlich, wenn einem weiteren Erfindungsmerkmal nach zur Bestimmung des verbleibenden chemischen Sauerstoffbedarfs (CSB) eine Fluoreszenzmessung herangezogen wird. Viele der eingeleiteten Stoffe fluoreszieren bei Bestrahlung durch ultraviolettes Licht. Insbesondere ist dies bei den für die Wasserqualität besonders wichtigen Kohlenwasserstoffen der Fall. Die Fluoreszenzmessung ergibt allerdings quantitativ eine Summenmessung aller im Abwasser mitgeführten fluoreszierenden Stoffe, was allerdings der Zuverlässigkeit des erfindungsgemäßen Verfahrens sehr entgegenkommt, da jetzt nicht nur eine einzige Indikatorsubstanz mit der gesamten Schmutzfracht verglichen wird, sondern eine Vielzahl solcher, im allgemeinen im Mischabwasser bereits vorhandenen Substanzen. Außerdem kann durch die Fluoreszenzmessung auch eine qualitative Bestimmung der mitgeführten Stoffe durch Fluoreszenzanregung mit Ultraviolettlicht unterschiedlicher Frequenz erreicht werden.

Selbstverständlich kann neben dem CSB auch, wie an sich bekannt, gleichzeitig der BSB₅ kontinuierlich bestimmt werden.

Der Einsatz des erfindungsgemäßen Verfahrens ist selbstverständlich auch dann möglich, wenn zur Reinigung des Abwassers nur eine Vorfällung erfolgt - also ohne eine Biostufe. Außerdem ist darauf hinzuweisen, daß nunmehr die Zugabe der verschiedenen Bakterien wie auch Fällungsmittel automatisiert werden kann, da jederzeit die Reinigungsleistung der Abwasser-Behandlungsanlage durch den Rest-CSB-Wert kontrolliert werden kann.

Die Anwendung des Verfahrens soll an Beispielen erläutert werden:
1. Das Rohabwasser eines Textilveredelungsbetriebes enthält neben Farbresten auch Öle, Kohlenwasserstoffe, Fette und Tenside. Die CSB-Gesamtbelastung liegt zwischen 2.000 und 3.500 mg/l bei einer Kohlenwasserstoffbelastung von etwa 60 mg/l. Nach einer Neutralisation mit Kalkmilch ergibt sich ein pH-Wert von 11, der nach Fällung mit FeIII und Pax + Polymere auf pH 7,2 zurückgeht. In der Vorfällung erfolgte bereits eine CSB-Reduzierung auf 240 mg/l und eine Kohlenwasserstoffreduzierung auf etwa 20 mg/l. Nach dem Einsatz einer Biostufe mit speziellen Bakterien wurde ein CSB-Abbau bis auf 60 mg/l bei einer Restbelastung von 5 mg/l Kohlenwasserstoffe erreicht. Die kontinuierliche Messung der Abwasserbehandlung erfolgte durch eine Fluoreszenzmessung der Kohlenwasserstoffe. Tatsächlich zeigte sich, daß der CSB-Abbau wie auch der Kohlenwasserstoff-Abbau parallel zueinander verlief, so daß, nachdem einmalig die Korrelation zwischen den fluoreszierenden Kohlenwasserstoffen und der Gesamtschmutzfracht festgestellt wurde, zusätzliche Messungen nicht erforderlich waren.
2. In gleicher Art und Weise wurde der CSB eines Spülabwassers aus einem Galvanikbetrieb bestimmt, wobei dem Spülabwasser Öle, Kohlenwasserstoffe, Fette, Tenside und Nitrite beigemengt waren, so daß sich beim Roh-Spülabwasser eine CSB-Gesamtbelastung von über 9.000 mg/l, eine Kohlenwasserstoffbelastung von etwa 120 mg/l und eine Nitritbelastung von bis zu 30 mg/l ergab. Auch hier wurde wiederum mit Kalkmilch neutralisiert und anschließend gefällt mit dem Resultat einer CSB-Reduzierung auf etwa 1.100 mg/l, einer Kohlenwasserstoffreduzierung auf etwa 40 mg/l und einer Nitritreduzierung auf 20 mg/l. Nach Zugabe von speziell adaptierten Bakterien ergab sich eine CSB-Reduzierung auf 130 mg/l, eine Kohlenwasserstoffreduzierung auf 15 mg/l und eine Nitritreduzierung auf etwa 5 mg/l. Auch hier verlaufen wieder die Kohlenwasserstoffabbauraten, wie Überprüfungen mit den üblichen diskontinuierlichen Verfahren ergaben, gleichlaufend mit der CSB-Abbaurate und etwa gleichlaufend mit der Nitritabbaurate, so daß eine Messung der Kohlenwasserstoffe, in diesem Falle wiederum mittels eines Fluoreszenzgerätes, die gewünschte kontinuierliche Erfassung des Rest-CSB zuließ.

## Patentansprüche

1. Verfahren zum Bestimmen des Chemischen Sauerstoff-Bedarfs (CSB) eines Mischabwassers mit einer Schmutzfracht aus organischen und annorganischen Substanzen durch Nachweis des verbleibenden CSB nach dem Durchlauf durch eine chemisch/physikalische und/oder biologische Abwasser-Reinigungsanlage,
dadurch gekennzeichnet, daß
- eine Indikatorsubstanz im Rohwasser ermittelt oder dem Rohwasser zugegeben wird, welche im Abbauverhalten weitgehend den gesamten im Rohwasser vorhandenen Stoffen entspricht,
- das Verhältnis des Abbaus der verschmutzenden Stoffe zum Abbau der Indikatorsubstanz ermittelt wird, und
- der Abbau der Indikatorsubstanz bestimmt wird.

2. Verfahren nach Anspruch 1,
dadurch gekennzeichnet,
daß die Indikatorsubstanzen dem Rohabwasser zugegeben werden.

3. Verfahren nach Anspruch 1 oder 2,
dadurch gekennzeichnet,
daß die Indikatorsubstanzen Kohlenwasserstoff-Verbindungen sind.

4. Verfahren nach Anspruch 3,
dadurch gekennzeichnet,
daß die Kohlenwasserstoff-Verbindung eine Aminocarbonsäure ist.

5. Verfahren nach Anspruch 1 oder 2,
dadurch gekennzeichnet,
daß die Indikatorsubstanz ein Xanthenfarbstoff ist.

6. Verfahren nach Anspruch 1,
dadurch gekennzeichnet,
daß die quantitative und/oder qualitative Erfassung der Indikatorsubstanzen mittels kontinuierlicher Fluoreszenzmessung erfolgt.

## Claims

1. A method of determining the chemical oxygen demand (C.O.D.) of a mixed sewage with a pollution load of organic and inorganic substances by detection of the remaining C.O.D. after passing through a chemical/physical and/or biological sewage cleaning plant, characterised in that
- an indicator substance is ascertained in the untreated water or is added to the untreated water, which substance substantially corresponds in decomposition characteristics to all the substances present in the untreated water,
- the relationship of decomposition of the pollutant substances to decomposition of the indicator substance is ascertained, and
- the decomposition of the indicator substance is determined.

2. A method according to claim 1 characterised in that the indicator substances are added to the untreated sewage.

3. A method according to claim 1 or claim 2 characterised in that the indicator substances are hydrocarbon compounds.

4. A method according to claim 3 characterised in that the hydrocarbon compound is an aminocarbonic acid.

5. A method according to claim 1 or claim 2 characterised in that the indicator substance is a xanthene dyestuff.

6. A method according to claim 1 characterised in that quantitative and/or qualitative detection of the indicator substances is effected by means of continuous fluorescence measurement.

## Revendications

1. Procédé de détermination de la demande chimique en oxygène (DCO) d'une eau résiduaire de mélange comportant une charge polluante formée de substances organiques et minérales, par détection de la DCO restante après le passage par une installation d'épuration physico-chimique et/ou biologique des eaux résiduaires,
procédé caractérisé en ce que :
- on détermine dans l'eau brute une substance indicatrice, ou on ajoute à l'eau brute une substance indicatrice, qui correspond dans une large mesure, dans leur comportement lors de la dégradation, aux matières totales présentes dans l'eau brute,
- on détermine le rapport entre la destruction ou la dégradation des matières polluantes et la destruction ou la dégradation de la substance indicatrice, et
- on détermine la destruction ou dégradation de la substance indicatrice.

2. Procédé selon la revendication 1, caractérisé en ce que les substances indicatrices sont ajoutées à l'eau résiduaire brute.

3. Procédé selon la revendication 1 ou 2, caractérisé en ce que les substances indicatrices sont des composés hydrocarbonés.

4. Procédé selon la revendication 3, caractérisé en ce que le composé hydrocarboné est un acide amino carboxylique.

5. Procédé selon la revendication 1 ou 2, caractérisé en ce que la substance indicatrice est un colorant de la série du xanthène.

6. Procédé selon la revendication 1, caractérisé en ce que la détermination quantitative et/ou qualitative des substances indicatrices est réalisée à l'aide d'une mesure en continu de la fluorescence.
